Europäisches Patentamt

European Patent Office

Office européen des brevets

(11) Publication number: **0 390 159 B1**

# EUROPEAN PATENT SPECIFICATION

(45) Date of publication of patent specification: **04.05.94**　(51) Int. Cl.5: **A61L 9/015**, F25D 17/04

(21) Application number: **90106052.5**

(22) Date of filing: **29.03.90**

(54) **Ozone deodorizing device.**

(30) Priority: **31.03.89 JP 83486/89**
　　　　　**31.03.89 JP 83487/89**
　　　　　**06.04.89 JP 87617/89**

(43) Date of publication of application:
**03.10.90 Bulletin 90/40**

(45) Publication of the grant of the patent:
**04.05.94 Bulletin 94/18**

(84) Designated Contracting States:
**CH DE FR GB IT LI NL SE**

(56) References cited:
**EP-A- 0 282 301**

(73) Proprietor: **MATSUSHITA ELECTRIC INDUSTRI-AL CO., LTD.**
**1006, Oaza Kadoma**
**Kadoma-shi, Osaka-fu, 571(JP)**

(72) Inventor: **Satou, Teruo**
**53-29, Kurigatani,**
**Hashimoto**
**Yawata-shi, Kyoto(JP)**
Inventor: **Mori, Iichiro**
**A11-102, 2-4-1, Korigaoka**
**Hirakata-shi, Osaka(JP)**
Inventor: **Watanabe, Takayuki**

**10-14, Yamanoue-kita-machi**
**Hirakata-shi, Osaka(JP)**
Inventor: **Sako, Yukinobu**
**4-30-8, Kisabe**
**Katano-shi, Osaka(JP)**
Inventor: **Fujita, Shigehiko**
**3-32-7, Miyanosaka**
**Hirakata-shi, Osaka(JP)**
Inventor: **Hatazaki, Yasuzou**
**2-21-18, Omiya,**
**Asahi-ku**
**Osaka-shi, Osaka(JP)**
Inventor: **Inoue, Yoshitaka**
**20-6, Wakaba-cho**
**Neyagawa-shi, Osaka(JP)**

(74) Representative: **Schwabe, Hans-Georg,**
**Dipl.-Ing. et al**
**Patentanwälte Schwabe, Sandmair, Marx**
**Stuntzstrasse 16**
**D-81677 München (DE)**

## Description

The present invention relates to an ozone deodorizing device adapted for use in refrigerators, and more particularly to an ozone deodorizing device for such use, operable or a battery without using a code. The codeless structure enables the device to be used easily in confined spaces such as refrigerators.

Ozone deodorizing devices operable by a commercial power source are known and used. These apparatus use a code connected to the power source. However, the code becomes an obstacle in confined spaces like refrigerators.

From EP 0 282 301 A3 a deodorizing apparatus is known which comprises a case with an air inlet and an air outlet and an ozone generator which is located at the air passage between the air inlet and the air outlet. The ozone generator comprises a dielectric layer and a pair of electrodes, one of which is situated at one side of the dielectric layer and the other is situated at its opposite side. A catalyst is also disposed within the air passage. A fan for effecting air circulation over the air passage is also disposed within the case of the known deodorizing apparatus. However, this kind of known deodorizing apparatus needs an external transformer in a transformer case which is connected to the national network because the known deodorizing apparatus needs a large voltage to guarantee an appropriate function over an acceptable time cycle. Therefore it will be necessary to mount further voltage inputs onto a refrigerator which should be provided with the known deodorizing apparatus.

To solve the above problems, codeless ozone deodorizing devices using a battery have been proposed. However, in return for the codeless structure, a voltage stabilizer circuit must be used so as to compensate for the discharge of the battery and maintain constant voltage to secure even corona discharge. Alternatively, a feedback is applied to the driving section by detecting the amount of corona discharge so as to equalize the amount of corona discharge.

A deodorizing device requires a d.c. motor for driving a fan for taking in and discharging air. It is necessary to check the condition of the motor constantly, and the common practice is to insert a d.c. resistance in the circuit of the motor so as to monitor any voltage drop. In addition, to check if the motor is in smooth rotation, a rotary detector is used. Furthermore, to prevent electronic components from becoming deteriorated owing to the leakage caused by over-discharge of the battery, a voltage cut-off circuit is used. The deodorizing device must induce an odorous air, and to this end the device is provided with a port through which air is passed.

The ozone deodorizing device of the present invention, which overcomes the above-discussed and numerous other disadvantages and deficiencies of the prior art, comprises a main body, an air passage extending from an intake port to an exhaust port, each port being formed in the wall of the main body, an ozone generator disposed in the passage, the ozone generator comprising a dielectric thin layer and a pair of electrodes, one of which is situated at one side of the dielectric thin layer and the other is situated at the opposite side thereof, a catalyst disposed downstream of the ozone generator to chemically change an odorous air into an odorless air and to decompose a surplus of ozone, a fan for effecting air circulation from the intake port to the exhaust port, a control mean for effecting the timely generation of ozone and the timely driving of the fan, and a d.c. battery power source for driving a motor for the fan.

In a preferred embodiment, the control circuit means comprises a delay feedback circuit for equalizing the varying amount of discharge, and a cut-off detecting circuit for detecting a leakage of the battery.

In a preferred embodiment, the control circuit means comprises means for detecting a disconnection in the motor and means for detecting a locking in the motor.

In a preferred embodiment, the ozone generator comprises a first timer for intermittently allowing the power source to discharge at predetermined time intervals, and a second timer for allowing the power source to discharge for a predetermined period of time by means of a start switch, the second timer being actuated at a desired time while the first timer is on.

In a preferred embodiment, a comparator is disposed in the control circuit means, the comparator effecting comparison by reference to predetermined threshold of a differentiating circuit, the output pulse of the comparator being subjected to width modulation by the delay characteristics of the delay feedback circuit.

In a preferred embodiment, the control circuit means comprises an R-S flip-flop, means for monitoring the terminal voltage of the power source, a first and a second integrating circuits, wherein the output decision signal from the monitoring means is applied to the R-S flip flop as a set input through the first integrating circuit and applied thereto as a reset input through the second integrating circuit.

In a preferred embodiment, the main body comprises at least one recess in which either the intake port or the exhaust port is formed so that the port in the recess is kept safe from enclosure by an obstacle in the refrigerator.

In a preferred embodiment, the intake port or the exhaust port not situated in the recess is separated by a spacer from a wall of the refrigerator.

In a preferred embodiment, the ozone deodorizing device includes a first lamp adapted to light up in response to the generation of ozone, the lamp being disposed in a front part of the deodorizing device, and a second lamp adapted to light up in response to the consumption of the power battery.

Thus, the invention described herein makes possible the objectives of (1) providing an ozone deodorizing device adapted for installation in a confined space because of the codeless structure, (2) providing an ozone deodorizing device capable of prolonging the life of a battery power source by minimizing the consumption of electricity, and (3) providing an ozone deodorizing device capable of strengthening the deodorizing power in accordance with the density of a smell.

This invention may be better understood and its numerous objects and advantages will become apparent to those skilled in the art by reference to the accompanying drawings as follows:

Figure 1 is a block diagram showing an example of the ozone deodorizing device according to the present invention;

Figure 2 is a circuit diagram including a creepage discharging circuit, incorporated in the ozone deodorizing device of Figure 1;

Figure 3 is a diagram showing a waveform of each driving circuit portion;

Figure 4 is a graph showing performances which modulate output pulse widths by an integrating circuit in the delay feedback circuit;

Figure 5 is a graph showing the relation between a source power and an output pulse width;

Figure 6 is a circuit diagram showing another example of the delay feedback circuit;

Figure 7 is a circuit diagram showing a further example of the delay feedback circuit;

Figure 8 is a diagram showing a circuit for detecting a disconnection in the d.c. motor

Figures 9 and 10 are diagrams showing modified versions of the disconnection detecting circuit;

Figure 11 is a diagram showing a lock detecting circuit in the d.c. motor;

Figure 12 is a graph showing a relation between the rpm of the d.c. motor and the terminal voltage;

Figure 13 is a diagram showing a cut-off detecting circuit;

Figure 14 is a front view showing the outward appearance of the ozone deodorizing device according to the present invention; and

Figure 15 is a cross-sectional side view showing the ozone deodorizing device.

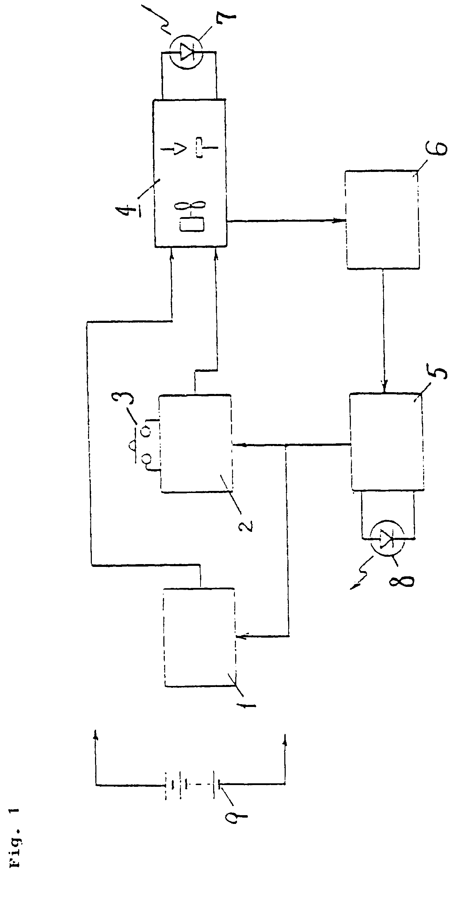

Referring to Figure 1, there is a first timer 1 which starts upon lapsing of a predetermined period of time and stops upon completion of a relatively short predetermined period of time. A second timer 2 is also provided, which is turned on by a switch 3, and stops upon completion of a relatively long predetermined period of time. Ozone is supplied from a supplier 4 which comprises a fan motor and an ozone generator circuit. The supply of ozone from the supplier 4 is controlled by the timers 1 and 2. The reference numeral 9 denotes a battery as a power source.

The smell in the refrigerator is normally not very strong and it is not difficult to remove it by operating the deodorizing device at regular time intervals so that the smell is prevented from becoming thick while the deodorizing device is at rest. In the illustrated embodiment the duty factor is roughly 0.03.

When the refrigerator contains foods having a strong smell such as unwrapped pickles or cheese, it is likely to be filled with a thick smell. In such cases, it is desirable to remove the smell as soon as possible. To achieve quick removal of smell, the second timer 2 is switched on to operate the deodorizing device so as to supply ozone continuously. In the illustrated embodiment the ratios of the period of time is 1 : 16 between the first timer 1 and the second timer 2. This shows that when the smell intensifies, it is removed quickly. The charge of the battery is monitored by a battery alarm circuit 5. If the capacity of the battery is consumed and the deodorizing performance decreases, the battery alarm lamp 8 is lit. Some batteries cause leakage owing to over-discharge, and the liquid is likely to damage electronic components inside the device and/or utensils outside it. The battery alarm circuit 5 immediately stops the first timer 1 and the second timer 2 when the battery is used up, and maintain the stoppage thereafter. In general, an ozone deodorizing device is designed to emit ozone in the exhaust which acts on odorants. The device is provided with a catalyst through which the mixture of ozone and odorants is expelled so that the content of ozone in the exhaust does not exceed a predetermined level. The fan guides the flow of the mixture, and if any failure occurs in the fan motor, the mixture will not flow through the prescribed route but be expelled outside the device without passing through the catalyst. In order to avoid such situations, a detector 6 is provided to detect if the fan motor stops, and if it finds that the fan motor is not in operation, it signals this to the alarm circuit 5 so as to light an alarm lamp, and stop the timers 1 and 2 immediately. Thus the supply of ozone is stopped. In the illustrated embodiment the deodorizing device is designed to inform an operator of such abnormalities.

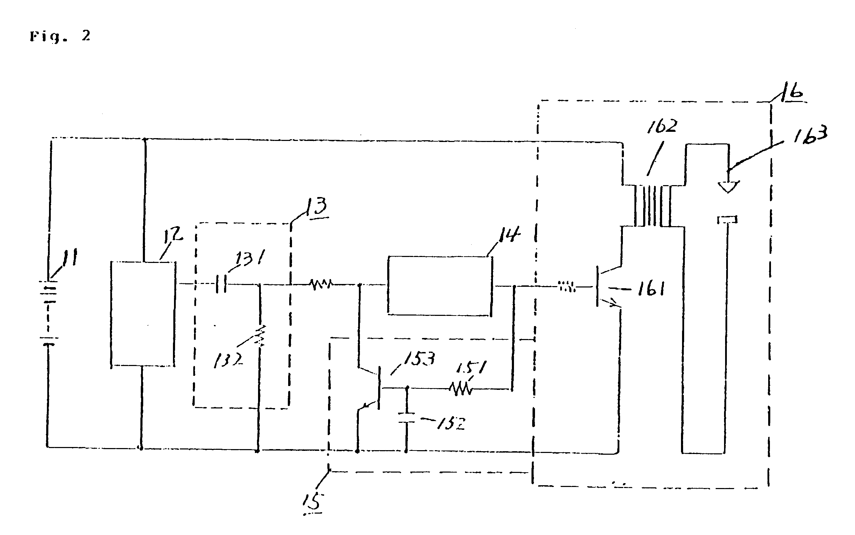

Referring to Figure 2, a driving circuit of a discharger will be described:

There are provided a battery 11, an oscillator 12 for generating rectangular waves, a differentiation circuit 13, such as a circuit having a capacitor 131, a resistance 132 connected in series. The reference numeral 14 denotes a comparator which selects outputs from the differentiation circuit 13 using a threshold, such as CMOS IC. The reference numeral 15 denotes a delay feedback circuit interposed between the input and the output of the comparator 14, the delay feedback circuit 15, for example, comprises a resistance 151 and a capacitor 152 connected to the output of the comparator 14, and an emitter grounded transistor 153 whose collector is connected to the input of the comparator 14. The reference numeral 16 denotes a creepage firing section which comprises a transistor 161 driven by the output of the comparator 14, a pulse transformer 162, and a creepage discharger 163. Since the transistor 161 is driven by the output of the comparator 14, the amount of energy stored in the pulse transformer 162 is determined by the current strength on the primary winding entirely depending on the source voltage and the width of output pulse of the comparator 14. The creepage firing section 16 is a free oscillator which is determined by the secondary winding of the pulse transformer 162 and the creepage discharger 163 itself. It fires when the energy stored in the pulse transformer 162 discharges.

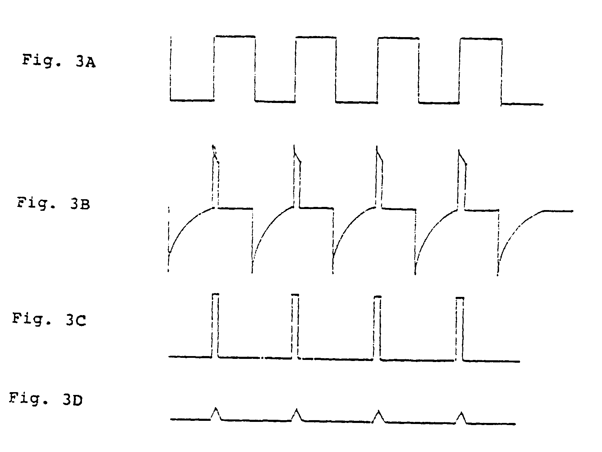

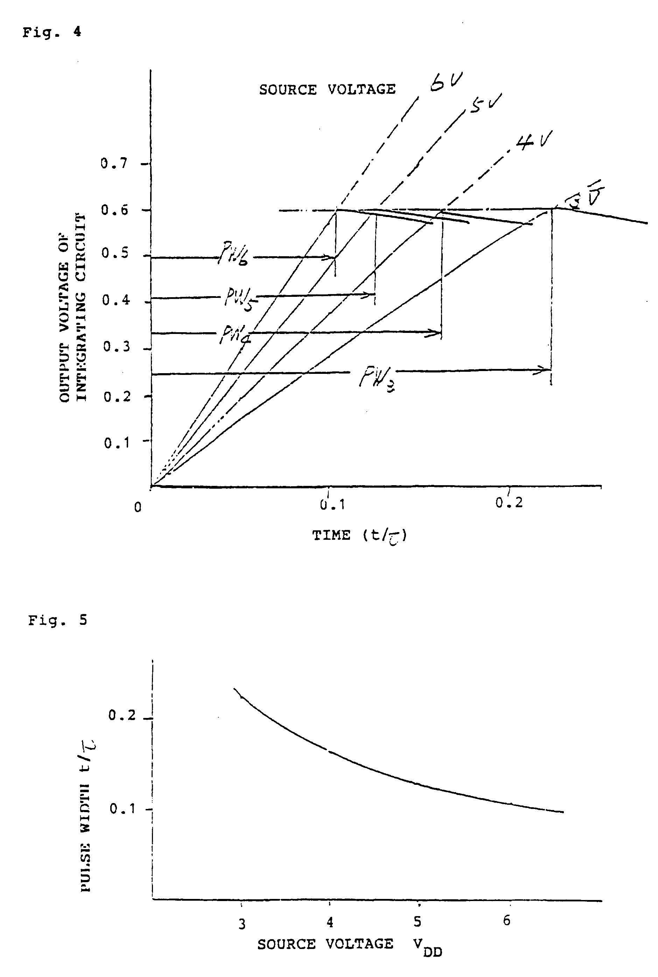

Referring to Figure 3 and Figure 4, the performance characteristics will be described, wherein Figure 3 is diagrams showing voltage waveforms of each driving circuit portion for driving creepage firing sections. Figure 3A shows an output waveform of the oscillator 12, Figure 3B shows an output waveform of the differentiation circuit 13; in other words, an input waveform of the comparator 14, Figure 3C shows an output waveform of the comparator 14, and Figure 3D shows a base voltage waveform of an emitter grounded transistor in the delay feedback circuit 15. Figure 4 is a graph plotting the output waveform, shown in Figure 3D, of the integrating circuit in the delay feedback circuit 15 in terms of a source voltage (6v to 3v) as a parameter.

When the output of the comparator 14 reaches "H" level, the output of the integrating circuit starts to rise at time constant $\tau$. The rising speed depends on the source voltage; more specifically, as shown in Figure 4, the higher the source voltage is, the higher speed is achieved. When the output voltage of the integrating circuit reaches roughly 0.6V the output of the differentiation circuit 13 rapidly drops, as shown in Figure 3B, because the transistor 153 is energized, and when it lowers below the threshold of the comparator 14, the output of the comparator 14 reaches "L" level. Thereafter, the output of the integrating circuit advances toward zero at the time constant $\tau$. The pulse widths of the comparator 14 become PW6, PW5, PW4 and PW3 corresponding to the 6V, 5V, 4V and 3V at the source voltage, respectively. As is evident from this relationship between the pulse width and the source voltage, the pulse widths expand with the decrease in the source voltage. The relation among the source voltage $V_{DD}$, the time constant $\tau$, the ON voltage $V_{BE}$ of a transistor 153 and the pulse width t is represented by

$$t/\tau = \ln\{V_{DD}/(V_{DD} - V_{BE})\}$$

this relation is shown in Figure 5.

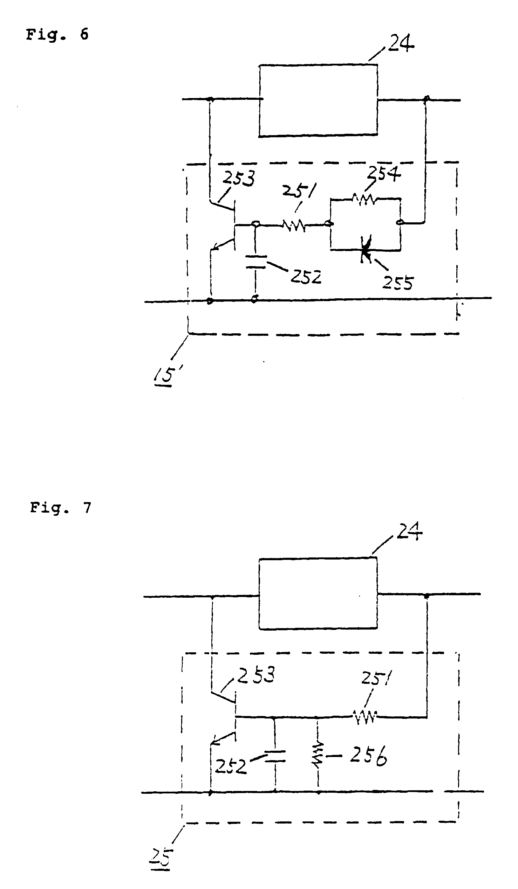

Figure 6 shows another example of the delay feedback circuit 15. In Figure 6 the integrating circuit 15′ includes a resisting component connected in series to a resistance 251 and a resistance 254 and a diode 255 in parallel. In this example the charging current of the capacitor 252 partly flows through the diode 255 at an initial stage, and the amount of current flow of current depends on the source voltage. More specifically, when the source voltage is high, more current flows through the diode 255 to the capacitor 252. As a result, the time constant of this integrating circuit cannot be constant but when the source voltage is high, the time constant is small and when it is low, the time constant is large. In contrast to the example of Figure 6, the graph of Figure 5 shows a case where the time constant of the integrating circuit is constant independently of the source voltage. The relation between the pulse width and the source voltage in the example of Figure 6 will be plotted with steeper curve. However if a Zener diode is used, the width of an output pulse of the comparator 14 (Figure 2) will be stabilized and become constant independently of the source voltage. Therefore it is possible to represent the relation between the pulse width and the source voltage in a more gradual curve than that of Figure 5 if any voltage-nonlinear device is employed to reduce the variations in the amplitude of output voltage of the comparator 24 occurring in accordance with changes of the power voltage. This is evident from the application of a Zener diode mentioned above.

Figure 7 shows a further modification of the delay feedback circuit 15. The modified circuit 25 is different from the example of Figure 2 in that it is additionally provided with a resistance 152 connected in parallel to the capacitor 252. In the example of Figure 7 the source voltage is divided by the resistances 151 and 256 and charges the capacitor 252. This is the same operation as that occurring when a lower part of the curve representing the relation between the source voltage and the output pulse width in Figure 5 is utilized. As a result, the resulting curve becomes steeper than that shown in Figure 5.

As described above, the intensity of the discharge of the creepage discharger can be controlled by varying the pulse width of the comparator 24. As a result, the amount of discharge can be stabilized by setting the relation between the source voltage and the output pulse by the delay feedback circuits 15, 15′ and 25 in accordance with the characteristics of the creepage discharger.

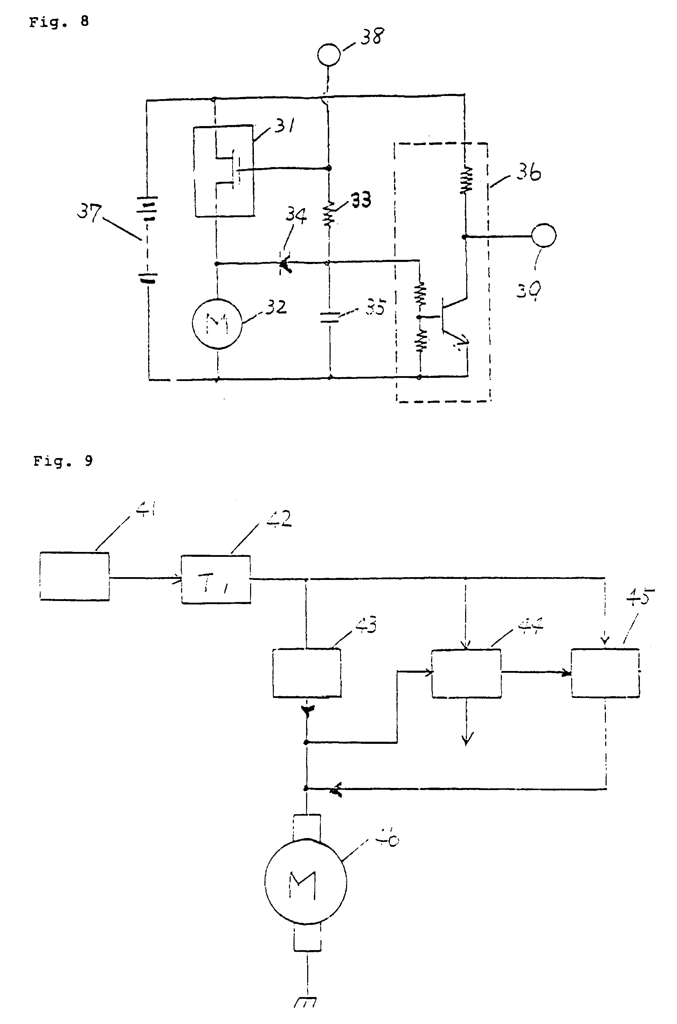

Referring to Figure 8, illustrating a disconnection detection circuit for detecting a possible disconnection in the d.c. motor of the present invention, the reference numeral 31, 32, 33, 34, 35, 36, 37, 38 and 39 denote a switch for turning on and off the d.c. motor 32, the d.c. motor, a resistance for supplying a small amount of current to the d.c. motor, a diode, a capacitor constituting the delay feedback circuit, a logic decision circuit for detecting the terminal voltage of the d.c. motor, a source battery, a control signal input terminal of the switching element 31, and a logic decision signal output terminal, respectively. The operation of the disconnection detection circuit will be described:

If a signal "LOW" is input to the control signal input terminal 38, the switching element will be on, thereby energizing the d.c. motor 32. At this stage the terminal 38 is kept at a level of "LOW", and a voltage at the positive side of the d.c. motor 32 is blocked by the diode 34 so that the voltage at the capacitor 35 is maintained at "LOW" level, and therefore the logic decision circuit 36 outputs a signal "HIGH" (meaning no abnormality occurs) to the logic decision signal output terminal 39. If a signal "HIGH" is given to the control signal input terminal 38, the switching element 31 will be off. Thereafter the d.c. motor 32 rotates by inertia, and generates a d.c. voltage between the terminals thereof. During this period of time the capacitor 35 is charged through the resistance 33 from the control signal input terminal 38, and the terminal voltage of the capacitor 35 rises at a time constant determined by the resistance 33 and the capacitor 35. When the d.c. motor 32 stops, the terminal voltage of the d.c. motor 32 becomes almost zero voltage because of the low resistance in the windings of the motor and the voltage in the capacitor 35 is clamped by a forward voltage of the diode 34. If the delay in the rising of the input signal at the logic decision circuit 36 by the delay circuit is set to be longer than a period of time required before the d.c. motor 32 stops upon damping of inertia, no input is effected to the logic decision circuit 36, thereby enabling its output terminal 39 to maintain the "HIGH" signal state. On the contrary, if the d.c. motor 32 has a disconnection, an infinitesimal current supplied to the motor will not be absorbed by the motor 32 through the diode 34, thereby causing the logic decision circuit 36 to output a signal "LOW".

As is evident from the foregoing description, while no disconnection occurs in the d.c. motor 32, the output of the logic decision circuit 36 remains "HIGH" not only when the control signal for the motor is "LOW" (i.e. the motor is on) but also when the control signal changes from "LOW" to "HIGH" (i.e. the motor 32 is switched from on to off). However, if a disconnection occurs in the motor 32, the output signal of the logic decision circuit 36 maintains "HIGH" when the control signal for the motor 32 is "LOW" (i.e. the motor is on) but if the control signal changes from "LOW" to "HIGH" (i.e. the motor 32 is switched from on to off), the output signal "LOW" is generated after a delay period of time provided by the delay circuit passes. It is possible to constitute the logic decision circuit 36 with logic elements such as CMOS-IC.

Figure 9 shows another modified detecting circuit for detecting a disconnection in the d.c. motor. In Figure 9 the reference numerals 41, 42, 43, 44, 45, and 46 denote a start circuit for generating start/stop signals to the motor: a timer circuit for determining a point of time at which it is checked if disconnection occurs when the motor is started, and turning off a first source circuit described below: a second source circuit for supplying an infinitesimal current through the d.c. motor at the point of time: a logic decision circuit for detecting a terminal voltage or a comparative voltage of the d.c. motor at the point of time: a first source circuit for driving the d.c. motor: and the d.c. motor, respectively.

The detecting circuit will be operated as follows:

The start circuit 41 generates a start signal in response to which the timer circuit 42 is energized at which the disconnection detecting time starts. At this stage the first source circuit 45 is kept off, supplying no current to the d.c. motor 46. At the same time the second source circuit 43 passes an infinitesimal current through the d.c. motor 46. However, the d.c. motor 46 does not rotate upon reception of the infinitesimal current from the second source circuit 43. As a result, the terminal voltage of the d.c. motor 46 amounts to the product of the infinitesimal current and a d.c. resistance component but the actual voltage is almost zero volts because these two components are negligibly small values.

When the point of detecting time is reached, and the logic decision circuit 44 detects that the terminal voltage of the d.c. motor 46 is almost zero, the circuit generates an "OK" signal. When the period of detecting time lapses upon completion of the timer circuit 42, the logic decision circuit 44 is latched and generates the "OK" signal, thereby turning on the first source circuit 45. In this way the d.c. motor 46 is put into operation.

When the d.c. motor 46 has a disconnection, the detecting circuit is operated as follows:

The timer circuit 42 starts in response to a start signal from the start circuit 41 at which the period of detecting time starts. At this stage the first source circuit 45 is off, thereby stopping the supply of a current to the d.c. motor 46. The second source circuit 43 supplies an infinitesimal current through the d.c. motor 46. However, because of the disconnection in the d.c. motor 46, the d.c. resistance will become infinite, and the terminal voltage will rise up to a release voltage of the source circuit 43. The logic decision circuit 44 detects that the terminal voltage is abnormally high and generates a "Abnormal" signal. Upon lapsing of the timer circuit 42 the first source circuit 45 and the second source circuit 43 are off, thereby suspending the detecting operation. At this stage the logic decision circuit 44 generates an "abnormal" signal.

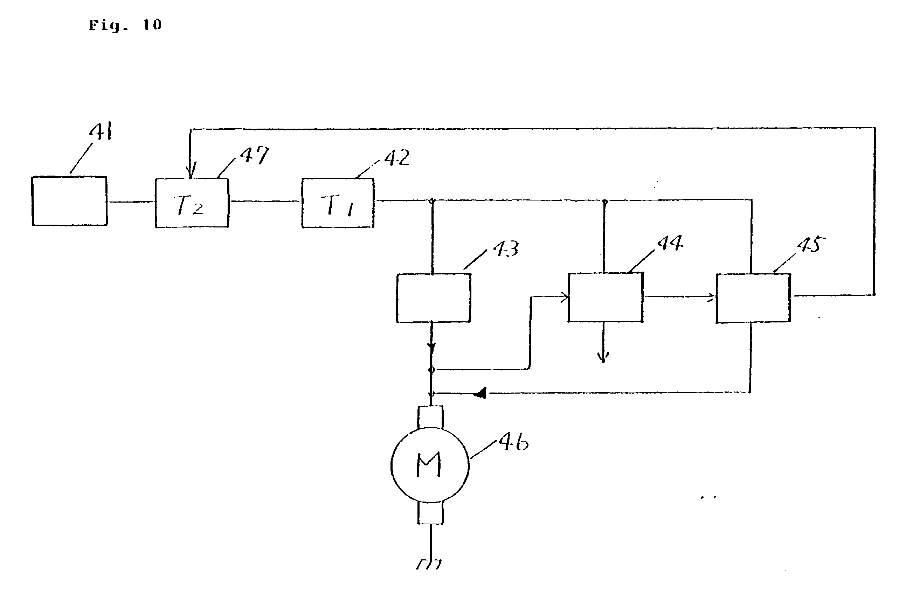

Figure 10 shows a further modification of the disconnection detecting circuit. In Figure 9 the reference numerals 41, 42, 43, 44, 45, 46 and 47 denote a start circuit for generating start/stop signals to the motor: a timer circuit for determining a point of time at which it is checked if disconnection occurs when the motor is started, and turning off a first source circuit described below: a second source circuit 43 for supplying a infinitesimal current through the d.c. motor at the point of time: a logic decision circuit for detecting a terminal voltage or a comparative voltage of the d.c. motor at the point of time: a first source circuit for driving the d.c. motor: and the d.c. motor, and a second timer circuit for determining a disconnection detection prohibiting time when the d.c. motor stops, respectively.

This modified disconnection detecting circuit is operated as follows:

The explanation will be given for a case where the d.c. motor stops, and the second timer circuit 47 does not work. In this case, the situation is virtually equal to when no second timer circuit 47 is employed.

When the first source circuit 45 becomes off and the d.c. motor 46 rotates by inertia, the d.c. motor 46 functions as a d.c. current generator, and generates a terminal voltage having an amplitude proportional to the rpm of the motor. At this stage, even if the start circuit 41 generates a start signal, as the second timer circuit 47 receives an off signal from the first source circuit 45, the second timer circuit 47 prohibits the disconnection detecting performance. As the inertia dampens owing to friction, the d.c. motor 46 stops and when the second timer circuit 47 is deenergized upon lapsing of the predetermined period of time, the start signal is transmitted to the first timer circuit 42. Then the first timer circuit 42 determines the disconnection detecting time, and the detecting operation starts. Thereafter, the same procedure as that shown Figure 9 follows.

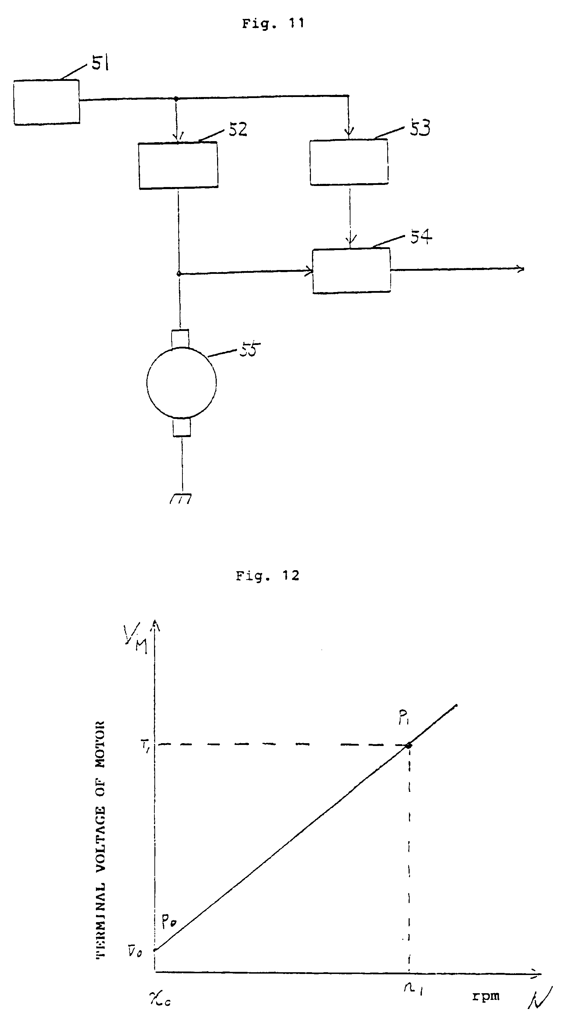

Figure 11 shows a lock detection circuit for checking if there is a locking in a d.c. motor 55. In Figure 11 the reference numerals 51, 52, 53, and 54 denote a start circuit for generating start/stop signals to the motor circuit; a d.c. source circuit for driving the d.c. motor and having a current limiting circuit; a timer circuit for setting a lock detection prohibiting time when the the d.c. motor starts; and a voltage comparator for detecting the terminal voltage of the d.c. motor. The lock detecting circuit is operated as follows:

When the start circuit 51 generates a start signal, the d.c. source circuit 52 and the timer 53 start. At this point of time the d.c. source circuit 52 drives the d.c. motor 55. The d.c. motor 55 speeds up from 0 rpm to a rated speed n1 rpm. The terminal voltage $V_M$ of the d.c. motor 55 is expressed by

$$V_M = KN + R_M \times I_M \quad (1)$$

where
K: time constant
$R_M$: d.c. resistance of the motor
$I_M$: a current flowing through the motor
N: rotations per minutes (rpm)

As shown in Figure 12, the d.c. motor starts from a point Po, and attains a stable speed at a point P1. The timer circuit 53, which is put into operation simultaneously with the source circuit 52, stops the voltage comparator 54 from detecting the terminal voltage of the motor for the lock detection prohibiting period of time prolonged until the the d.c. motor 55 reaches the stable point P1. Then the timer circuit 53 comes into operation, thereby releasing the lock detection prohibiting period of time. At this point of time the voltage comparator 54 starts. At this stage the d.c. motor 55 is in stable operation, and the voltage comparator 54 generates an "OK" signal. Thereafter, if the d.c. motor 55 is subjected to locking, the terminal voltage $V_{ML}$ is expressed by

$$V_{ML} = R_M \times I_M \quad (2)$$

where the d.c. resistance $R_M$ of the motor 55 is negligibly small, and the current $O_M$ through the motor is determined by a current value limited by the d.c. source circuit 52. As a result, the terminal voltage of the motor 55 is relatively small. The voltage comparator 54 detects the locking voltage $V_{ML}$ of the motor 55, and generates a motor locking signal. It is possible to constitute the voltage comparator 54 with logic elements such as CMOS-IC.

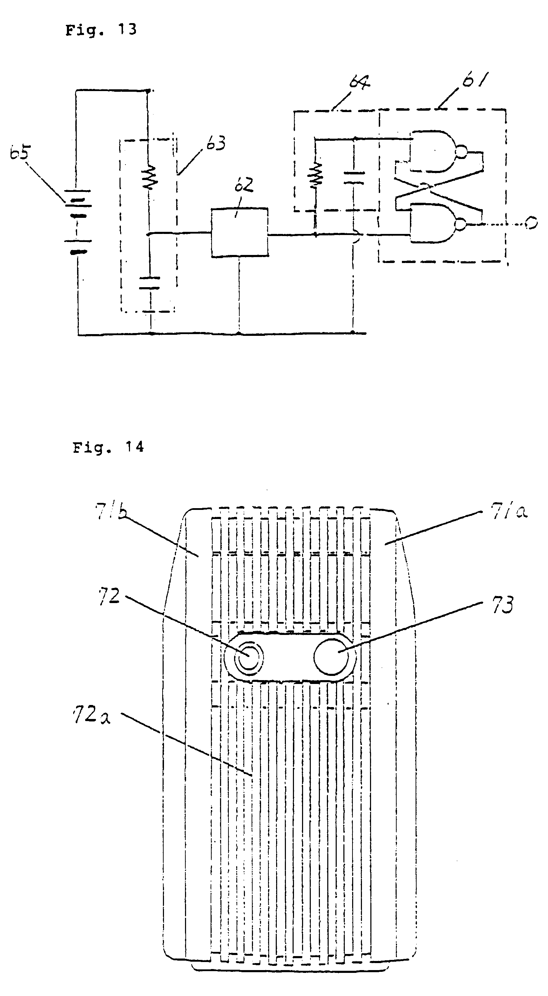

Figure 13 shows a cut-off detecting circuit for the battery. The reference numeral 61 denotes an R-S flip-flop employing a NAND gate to which an output of a voltage detecting element 62 is applied as a setting input signal. A setting input signal is also applied to a reset input terminal through a second integrating circuit 64. The voltage of the battery 65 is connected to an input terminal of the voltage detecting element 62 through a first integrating circuit 63. When a normal voltage is applied to the cut-off detecting circuit from the battery, the application is delayed by the first integrating circuit 63, and the applied voltage is compared with a reference voltage by the voltage detecting element 62 to generate a decision signal "H" level, which is applied to a set input terminal of the R-S flip-flop 61. This signal is also applied to a reset terminal of the R-S flip-flop through the second integrating circuit 64. As a result, the reset terminal changes from "L" level to "H" level later than the set terminal, thus completing the power-on-reset function. In this case, the function of the first integrating circuit 63 is to prevent a malfunction resulting from an instantaneous disconnection in a line leading to the battery. This function is performed regardless of whether the first integrating circuit 63 may be inserted at either side of the input terminal or an output terminal.

The cut-off detecting performance will be described:

The terminal voltage gradually drops with the consumption of the battery. When the load on the battery intermittently varies because of the internal resistance of the battery, the drop of the terminal voltage increases when the load power is large. While the load power is large, the terminal voltage of the battery drops below the reference voltage of the voltage detecting element 62. At this stage the output level of the voltage detecting element 62 changes from "H" level to "L" level. Thus the flip-flop is set to generate a cut-off signal in response to which the performance is stopped for the period of time when the load power is large, thereby causing the load power to change to a lower level instantly. In this way the terminal voltage of the battery rapidly rises. The output of the voltage detecting element 62 regains the "H" level. The set input becomes a pulse having a short width, and is not transmitted to the reset input terminal of the R-S flip-flop 61 because of the presence of the second integrating circuit 64, thereby enabling the R-S flip-flop to maintain its setting condition. Since the R-S flip-flop 61 and the voltage detecting element 62 can be constituted by CMOS type elements, the consumption of electric power on the detecting circuit is negligibly small. In addition, since a load power to the main circuit can be stopped by the cut-off detecting signal, the consumption of electricity is minimized after the cut-off detection is performed.

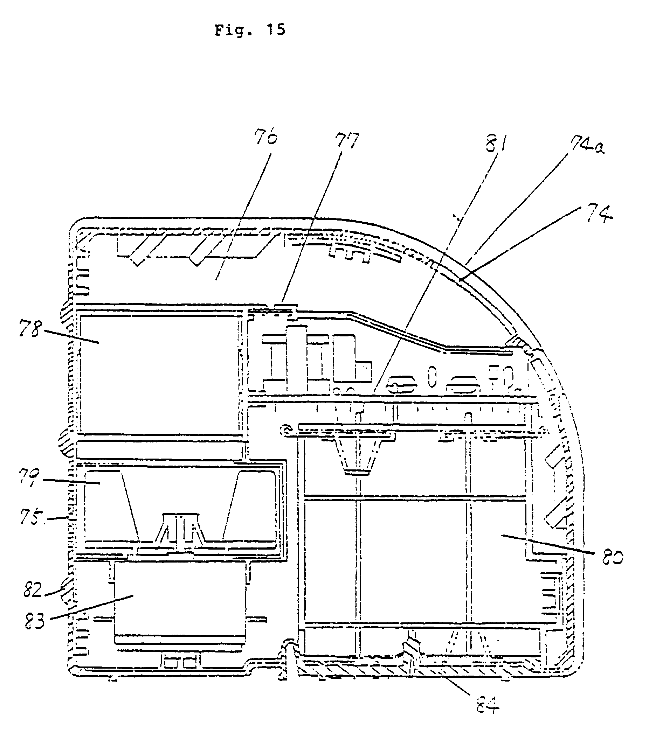

Referring to Figures 14 and 15, the ozone deodorizing device will be described:

The ozone deodorizing device is provided with a casing which consists of halved parts 71a and 71b. The reference numeral 72 denotes a display lamp disposed on a cover 72a which is lit when the device is in operation with the timer on. The operator can observe it. The reference numeral 73 denotes a button for immediately starting the deodorizing device. By pressing this button the second timer is forced into operation, and the device is put into operation a longer period of time than the period of time when the first timer keeps the "on" conditions. Thus the odorant is expelled out of the refrigerators. The reference numeral 74 denotes an intake port which is disposed flush with a portion 74a having the same curvature as that of the cover 72a. The reference numeral 75 denotes an exhaust port to which air introduced through the intake port 74 is led by way of the passage 76. Ozone is generated by a creepage discharger 77, which is situated at such a high place in the passage 76 from the bottoms of the intake port 74 or exhaust port 75 as to avoid water droplets accumulating on the discharger. Preferably, the discharger 77 is fitted in a groove produced in either of the halved parts 71a or 71b and held by the other halved part. The reference numeral 78 denotes a honeycomb shaped catalyst which decomposes odorants and reduces a remaining ozone to oxygen. The reference numeral 79 denotes a fan which induces air through the intake port 74 and expels it through the exhaust port 75. The battery is housed in a case 80. The intermittent generation of ozone and the fan 79 are controlled by a control circuit 81. The reference numeral 82 denotes projecting spacers whereby the exhaust port 75 is prevented from being closed by a structure (not shown) such as the inside wall of the refrigerator. The fan 79 is driven by a motor 83. The battery is loaded and unloaded by opening a lid 84.

The ozone deodorizing device is operated as follows:

The battery source is loaded and the control circuit 81 operates. The creepage discharger 77 generates ozone. At this stage the motor 83 is started, to drive the fan 79, thereby inducing the air in the refrigerator into the deodorizing device through the intake port 74. The air is induced through the intake port 74 has a stinking odorants. The air is forced through the honeycomb catalyst 78, and during the passage the odorant is decomposed into a substance having no substantial smell. The surplus of ozone is reduced to oxygen, and the deodorized air is discharged through the exhaust port 75. Since the intake port 74 is produced on the curved portion 74a it is not obstructed by an inside wall of the refrigerator or any other obstruct. The exhaust port 75 is kept open from a structure of the refrigerator by the projecting spacers 82. Alternatively, it is possible to produce the exhaust port 75 on a curved surface, and produce the projecting spacers 82 adjacent to the intake port 74. In this way the intake port 74 and the exhaust port 75 are kept free from any obstacle so that the air is smoothly circulated through the refrigerator. When the smell is especially strong or thick, the actuation of the rapid deodorizing switch prolongs the deodorizing period of time so that the strong smell is substantially removed. In other to prevent the creepage discharger from being contaminated from broth dripping from foods or any other liquid, it is situated at such a higher place in the passage of air. The creepage discharger is fitted in the groove of one of the halved case portion and held by the other case portion in such a manner as to be safe from becoming loose owing to shock. The outward appearance of the deodorizing device is simplified, and compact to that it can be installed in a limited space.

It is understood that various other modifications will be apparent to and can be readily made by those skilled in the art without departing from the scope and spirit of this invention. Accordingly, it is not intended that the scope of the claims appended hereto be limited to the description as set forth herein, but rather that the claims be construed as encompassing all the features of patentable novelty that reside in the present invention, including all features that would be treated as equivalents thereof by those skilled in the art to which this invention pertains.

## Claims

1. An ozone deodorizing device for use in a refrigerator, the device comprising a main body (71a, 71b), an air passage (76) extending from an intake port (74) to an exhaust port (75), each port being formed in the wall of the main body, an ozone generator (77) disposed in the passage, the ozone generator comprising a dielectric thin layer and a pair of electrodes, one of which is situated at one side of the dielectric thin layer and the other is situated at the opposite side thereof, a catalyst (78) disposed downstream of the ozone generator (77) to chemically change an odorous air into an odorless air and decompose a surplus of ozone, a fan (79) for effecting air circulation from the intake port (74) to the exhaust port (75), a control means for effecting the timely generation of ozone and the timely driving of the fan (7), and a d.c. battery power source for driving a motor (32, 83) for the fan (79).

2. An ozone deodorizing device according to claim 1, wherein the control circuit means comprises a delay feedback circuit for equalizing the varying amount of discharge, and a cut-off detecting circuit.

3. An ozone deodorizing device according to claim 1, wherein the control circuit means comprises means for detecting a disconnection in the motor and means for detecting a locking in the motor.

4. An ozone deodorizing device according to claim 1, wherein the ozone generator comprises a first timer for intermittently allowing the power source to discharge at predetermined time intervals, and a second timer for allowing the power source to discharge for a predetermined period of time by means of a start switch, the second timer being actuated at a desired time while the first timer is on.

5. An ozone deodorizing device according to claim 2, further comprising a comparator in the control circuit means, the comparator effecting comparison using predetermined threshold of a differentiating circuit, the output pulse of the comparator being subjected to width modulation by the delay characteristics of the delay feedback circuit.

6. An ozone deodorizing device according to claim 2, wherein the control circuit means comprises an R-S flip-flop, means for monitoring the terminal voltage of the power source, a first and a second integrating circuits, wherein the output decision signal from the monitoring means is applied to the R-S flip flop as a set input through the first integrating circuit and applied thereto as a reset input through the second integrating circuit.

7. An ozone deodorizing device according to claim 1, wherein the main body comprises at least one recess in which either the intake port or the exhaust port is formed so that the port in the recess is kept safe from enclosure by any obstacle in the refrigerator.

8. An ozone deodorizing device according to claim 1, wherein the intake port or the exhaust port not situated in the recess is spaced by a spacer from the wall of the refrigerator.

9. An ozone deodorizing device according to claim 1, further comprising a first lamp adapted to light up in response to the generation of ozone, the lamp being disposed in a front part of the deodorizing device, and a second lamp adapted to light up in response to the consumption of the power battery.

**Patentansprüche**

1. Vorrichtung zum Deodieren mit Ozon zur Verwendung in einem Kühlgerät bzw. Kühlschrank, wobei die Vorrichtung einen Grund- bzw. Hauptkörper (71a, 71b), einen Luftdurchgang (76), der sich von einer Einlaßöffnung (74) zu einer Abgasöffnung (75) erstreckt, wobei jede Öffnung in der Wand des Hauptkörpers ausgebildet ist, und einen Ozongenerator (77), der in dem Durchgang angeordnet ist, aufweist, wobei der Ozongenerator aufweist: eine dielektrische Dünnschicht und ein Paar Elektroden, von denen eine an einer Seite der dielektrischen Dünnschicht vorgesehen ist und die andere an der anderen Seite davon vorgesehen ist, einen Katalysator (78), der stromabwärts des Ozonenerators (77) angeordnet ist, um chemisch eine geruchsbeladene Luft in eine geruchlose Luft auszutauschen und einen Überschuß an Ozon abzubauen, einen Lüfter bzw. Ventilator (79), um eine Luftzirkulation von der Einlaßöffnung (74) zu der Abgasöffnung (75) zu bewirken, eine Steuereinrichtung, um die zeitliche Erzeugung von Ozon und das zeitliche Antreiben des Lüfters bzw. Ventilators (7) zu bewirken, und einer DC-Batterieenergiequelle zum Antreiben eines Motors (32, 83) des Lüfters bzw. Ventilators (7).

2. Vorrichtung zum Deodorieren mit Ozon nach Anspruch 1, in der die Steuerschaltungseinrichtung eine Verzögerungs-Rückkopplungs-Schaltung, um die sich verändernde Größe der Entladung auszugleichen, und eine Ausschaltdetektorschaltung aufweist.

3. Vorrichtung zum Deodorieren mit Ozon nach Anspruch 1, in der die Steuerschaltungseinrichtung Mittel zur Detektion einer Abschaltung des Motors und Mittel zur Detektion eines Sperrens des Motors aufweist.

4. Vorrichtung zum Deodorieren mit Ozon nach Anspruch 1, in der der Ozongenerator einen ersten Zeitgeber zur intermittierenden bzw. diskontinuierlichen Entladung der Energiequelle zu vorgegebenen Zeitintervallen, und einen zweiten Zeitgeber aufweist, um sich über eine vorgegebene Zeitperiode mittels eines Startschalters zu entladen, wobei der zweite Zeitgeber zu einer gewünschten Zeit betätigbar ist, während der erste Zeitgeber an ist.

5. Vorrichtung zum Deodorieren mit Ozon nach Anspruch 2, die ferner einen Vergleicher in der Steuerschalteinrichtung aufweist, wobei der Vergleicher einen Vergleich bewirkt, wobei der vorbestimmte Schwellwert einer Differentialschaltung verwendet wird, wobei der Ausgangsimpuls des Vergleichers einer Breitenmodulation durch die Verzögerungscharakteristiken der Verzögerungs-Rückkopplungs-Schaltung unterzogen wird.

6. Vorrichtung zum Deodorieren mit Ozon nach Anspruch 2, in der die Steuerschalteinrichtung einen R-S-Flip-Flop, Mittel zur Erkennung der Pol- bzw. Anschlußspannung der Energiequelle, und eine erste und eine zweite Integrationsschaltung aufweist, in der das Ausgangsentscheidungssignal der Erkennungseinrichtung dem R-S-Flip-Flop als eine Eingangseinstellung durch die erste Integrationsschaltung zugeführt wird und daran als Rücksetzeingangswert durch die zweite Integrationsschaltung angelegt wird.

7. Vorrichtung zum Deodorieren mit Ozon nach Anspruch 1, in der der Hauptkörper zumindest eine Ausnehmung aufweist, in der entweder die Einlaßöffnung oder die Abgasöffnung ausgebildet ist, so daß die Öffnung in der Ausnehmung durch die Einfassung vor Störungen in dem Kühlaggregat bzw. Kühlschrank in Sicherheit ist.

8. Vorrichtung zum Deodorieren mit Ozon nach Anspruch 1, in der die nicht in der Ausnehmung vorgesehene Einlaßöffnung oder Abgasöffnung durch einen Abstandhalter von der Wand des Kühlgeräts bzw. des Kühlschranks beabstandet ist.

9. Vorrichtung zum Deodorieren mit Ozon nach Anspruch 1, die ferner eine erste Lampe, die angepaßt ist, um in Abhängigkeit zur Erzeugung von Ozon aufzuleuchten, wobei die Lampe an einem Frontteil der Vorrichtung zum Deodorieren angeordnet ist, und eine zweite Lampe aufweist, die angepaßt ist, um in Abhängigkeit zum Verbrauch der Batterieenergie aufzuleuchten.

## Revendications

**1.** Un dispositif désodorisant à l'ozone destiné à être utilisé dans un réfrigérateur, le dispositif comprenant un corps principal (71a, 71b), un passage d'air (76) s'étendant d'un orifice d'entrée (74) à un orifice de sortie (75), chaque orifice étant ménagé dans la paroi du corps principal, un générateur d'ozone (77) disposé dans le passage, le générateur d'ozone comprenant une couche diélectrique mince et une paire d'électrodes dont une est située sur l'un des côtés de la couche diélectrique mince et l'autre est située sur le côté opposé de ladite couche, un catalyseur (78) disposé en aval du générateur d'ozone (77) pour transformer chimiquement un air odorant en un air inodore et pour décomposer un excédent d'ozone, un ventilateur (79) pour provoquer une circulation d'air de l'orifice d'entrée (74) vers l'orifice de sortie (75), un moyen de commande pour provoquer la production en temps d'ozone et l'actionnement en temps du ventilateur (7), et une source de courant continu à pile pour entraîner un moteur (32, 83) destiné au ventilateur (79).

**2.** Un dispositif désodorisant à l'ozone selon la revendication 1, caractérisé en ce que le moyen formant circuit de commande comprend un circuit de rétroaction à retard pour égaliser la quantité variable de décharge, et un circuit de détection de coupure.

**3.** Un dispositif désodorisant à l'ozone selon la revendication 1, caractérisé en ce que le moyen formant circuit de commande comprend un moyen pour détecter une déconnexion dans le moteur, et un moyen pour détecter un blocage dans le moteur.

**4.** Un dispositif désodorisant à l'ozone selon la revendication 1, caractérisé en ce que le générateur d'ozone comprend une première minuterie pour permettre par intermittence à la source de courant de se décharger à des intervalles de temps prédéterminés, et une seconde minuterie pour permettre à la source de courant de se décharger pendant une période de temps prédéterminée au moyen d'un commutateur de déclenchement, la seconde minuterie étant actionnée à un moment désiré pendant que la première minuterie est en marche.

**5.** Un dispositif désodorisant à l'ozone selon la revendication 2, comprenant également un comparateur implanté sur le moyen formant circuit de commande, le comparateur effectuant une comparaison à l'aide d'un seuil prédéterminé d'un circuit différenciateur, l'impulsion de sortie du comparateur étant soumise à une modulation en largeur par les caractéristiques de retard du circuit de rétroaction à retard.

**6.** Un dispositif désodorisant à l'ozone selon la revendication 2, caractérisé en ce que le moyen formant circuit de commande comprend une bascule électronique R-S, un moyen pour surveiller la tension aux bornes de la source de courant, un premier et un second circuit d'intégration, le signal de décision de sortie délivré par le moyen de surveillance étant appliqué à la bascule électronique R-S en tant qu'entrée à un à travers le premier circuit d'intégration et y étant appliqué en tant qu'entrée de remise à zéro à travers le second circuit d'intégration.

**7.** Un dispositif désodorisant à l'ozone selon la revendication 1, caractérisé en ce que le corps principal comprend au moins un évidement dans lequel est réalisé soit l'orifice d'entrée soit l'orifice de sortie, de façon que l'orifice placé dans l'évidement ne puisse pas être obstrué par un obstacle situé dans le réfrigérateur.

**8.** Un dispositif désodorisant à l'ozone selon la revendication 1, caractérisé en ce que l'orifice d'entrée ou l'orifice de sortie non situé dans l'évidement est séparé par un moyen d'écartement de la paroi du réfrigérateur.

**9.** Un dispositif désodorisant à l'ozone selon la revendication 1, comprenant également une première lampe conçue pour s'allumer en cas de production d'ozone, la lampe étant disposée dans la partie avant du dispositif désodorisant, et une seconde lampe conçue pour s'allumer en cas de consommation de la pile d'alimentation.

Fig. 1

Fig. 2

EP 0 390 159 B1

Fig. 3A

Fig. 3B

Fig. 3C

Fig. 3D

**Fig. 4**

**Fig. 5**

Fig. 6

Fig. 7

Fig. 8

Fig. 9

16

Fig. 10

Fig. 11

Fig. 12

Fig. 13

Fig. 14

Fig. 15